(19) Europäisches Patentamt

European Patent Office

Office européen des brevets

(11) Publication number: **0 288 145**
**A1**

(12) # EUROPEAN PATENT APPLICATION

(21) Application number: 88302322.8

(22) Date of filing: 17.03.88

(51) Int. Cl.⁴: **A61M 1/10 , A61M 1/14 , A61M 1/00 , F04B 43/00**

(30) Priority: 17.03.87 AU 939/87

(43) Date of publication of application:
**26.10.88 Bulletin 88/43**

(84) Designated Contracting States:
**AT BE CH DE ES FR GB GR IT LI LU NL SE**

(71) Applicant: **Logan, Kenneth Arthur**
**5 Banks Avenue**
**Hampton Victoria 3188(AU)**

(72) Inventor: **Logan, Kenneth Arthur**
**5 Banks Avenue**
**Hampton Victoria 3188(AU)**

(74) Representative: **Pidgeon, Robert John**
**Appleyard Lees 15 Clare Road**
**Halifax HX1 2HY(GB)**

(54) **Disposable pump element for dialysis pump.**

(57) A disposable pump element (4) comprising a moulded plastics body (30) formed with a pumping chamber (31) having a pair of valve housing (32, 33) into which inlet and outlet passages (34, 35) open. The pumping chamber (31) is hermetically sealed by means of a flexible plastics wall (38) heat sealed to the face of the body (30) around the pumping chamber (31). One way valves (36, 37) are received in the valve housings (32, 33) to allow the passage of fluid, such as blood, into and out of the chamber (31) in one direction only. The pump element body (30) is also moulded with a pressure sensing chamber (39) closed by a flexible plastics wall (40) heat sealed to the body (30) around the chamber (39). Inlet and outlet passages (41, 42) are formed in the body (30) and open into the chamber (39). Plastics tubing (T) connects the inlet passage (34) to the patient, the outlet passage (35) to a dialysis machine, the outlet of which is connected to the inlet passage (41) to the pressure chamber (39), the outlet passage (42) being connected by tubing (T) to the patient.

FIG. 5.

EP 0 288 145 A1

## DISPOSABLE PUMP ELEMENT FOR DIALYSIS PUMP

### FIELD OF THE INVENTION

This invention relates to improvements in pumps suitable for use as a means for pumping blood, for example in dialysis pumps, and more particularly to an improved disposable pump element for a dialysis pump.

### BACKGROUND OF THE INVENTION

In most clinical applications requiring the external circulation of blood, peristaltic pumps are widely used. In operations in which the blood must be pumped for long periods, such liver transplant operations or plasma exchange operations, peristalitc pumps are not suitable since long term use causes damage to the red blood cells. At the present time, long term blood circulation is achieved by the use of centrifugal pumps, but such pumps have the disadvantage of being extremely expensive.

In United States patent 4410322, Archibald, there is disclosed a non-pulsating IV pump having a plurality of flexible rolling diaphragm pumping chambers formed in a disposable assembly. A plurality of pistons act on the pumping chambers to discharge the contents of the chambers from one to the other and eventually to an outlet tube. Valves are provided to ensure that the fluid being pumped moves in the direction of the outlet tube only. The pump described in the patent is directed towards the administration of intravenous fluids and the patent does not envisage the pump being used in the pumping of.blood. One significant disadvantage of the pump described in this patent is the direct contact between the pistons and each of the flexible rolling diaphragm pumping chambers which may give rise to damage to one or more of the chambers with consequential loss or contamination of the fluid contents.

Another disadvantage of the pump described in the above United States patent is that the disposable assembly does not facilitate the direct measurement of the pressure of the fluid being pumped. In the case of dialysis pumps, it will be appreciated that it is important to know the pressure of the blood being pumped for dialysis treatment. Most currently available dialysis pumps, such as the Gambro dialysis pump, incorporate a pressure monitor which measures the pressure of the fluid in the system at the position of a bubble trap in the system, and with this arrangement it is necessary to have a monitor isolator positioned between the line to the bubble trap and the pressure monitor to ensure isolation of the fluid system. Since avoidance of loss or contamination of the blood in the system is extremely important, it is highly desirable that both the pumping and pressurement measurement should take place within a completely closed system which does not require the intervention of isolation means.

United States Patent 4468222 Lundquist, also discloses a pumping system having a disposable pump body, which differs from the Archibald arrangement in that the pump is activated by a flexible membrane. While this arrangement overcomes the direct contact problem of Archibald, the pump still does not have a means for monitoring pressure which is part of the closed system of the pump.

### SUMMARY OF THE INVENTION AND OBJECTS

It is an object of the present invention to provide an improved disposable pump element suitable for use in a dialysis pump in which both the fluid pumping and pressure measurement may be achieved as part of a completely closed system.

It is a subsidiary object of the present invention to provide a dialysis pump suitable for use with the disposable pump element of the present invention by means of which long term pumping of blood may be achieved while avoiding the disadvantages of the prior art pumps to thereby provide an acceptable alternative to a peristaltic pump.

Accordingly, the invention provides a disposable pump element for a pumping system comprising a pumping chamber closed by a flexible or displaceable wall, inlet and outlet passages connected to said chamber, a pressure monitoring chamber closed by a flexible or displaceable wall, and inlet and outlet passages opening into said monitoring chamber, said outlet passage of said pumping chamber being connectable to said inlet passage of said monitoring chamber so that the pressure of the fluid leaving said pumping chamber is able to be monitored through said wall closing said monitoring chamber while maintaining the fluid being pumped within a closed system.

It will be appreciated that the pump element according to the invention provides not only a means by which fluid such as blood may be pumped within a closed system, whereby loss and contamination is prevented, but also provides, as part of the same closed system, a direct means of measuring the pressure of the fluid being pumped. This avoids the need for intervening isolators and provides a relatively inexpensive means of performing contamination free pumping and pressure measurement.

In a preferred form of the invention, the pumping and pressure monitoring chambers are formed in a relatively rigid plastics moulding, said chambers being closed by one or more sheets of flexible plastics film secured to said moulding to cover said chambers.

In an alternative form of the invention, the pump element comprises a pair of sheets of plastics film sealed together to define said pumping and pressure monitoring chambers and said inlet and outlet passages, said inlet and outlet passages being adapted to receive plastics tubing for connection to a system providing and receiving fluid and for connecting said outlet passage of said pumping chamber to said inlet passage of said monitoring chamber.

In a particularly preferred form of the invention, the inlet and outlet passages to said pumping chamber include one way valves which prevent flow of fluid in the wrong direction.

In another aspect, the invention provides a pumping system for blood and other fluids comprising pumping means for causing generally pulsatile deflection of said flexible or displaceable wall closing the pumping chamber of the disposable pump element defined above to cause displacement of fluid contained in said pumping chamber through said outlet passage and into said pressure monitoring chamber, and pressure monitoring means positioned to be held in contact with said flexible or displaceable wall of said pressure monitoring chamber to sense the pressure of the fluid contained in said monitoring chamber, said pumping system including means for detachably securing said disposable pump element with its flexible or displaceable walls in contact with said pumping and pressure monitoring means.

In one preferred form of the invention, said pumping means comprises a plate mounted for reciprocating movement into and out of said pumping chamber in contact with said flexible or displaceable wall. The plate is preferably covered by a flexible diaphragm to reduce the risk of damage to the flexible wall and to facilitate cleaning and avoid contamination of the pumping system.

## BRIEF DESCRIPTION OF THE DRAWINGS

Two presently preferred embodiments of the invention will now be described with reference to the accompanying drawings in which:

Figure 1 is a perspective view of a pump embodying the invention;

Figure 2 is a fragmentary plan view of part of the pump of figure 1;

Figure 3 is a sectional elevation of the same part of the pump taken along the line 3-3 in figure 2;

Figure 4 is a perspective view from beneath of a disposable pump element embodying the invention for use with the pump of figures 1 to 3;

Figure 5 is a sectional plan view of the pump element of figure 4;

Figure 6 and 7 are sectional end elevations of the pump element taken along the lines 6-6 and 7-7 respectively; and

Figure 8 is a plan view of an alternative form of the pump element embodying the invention.

## DESCRIPTION OF PREFERRED EMBODIMENTS

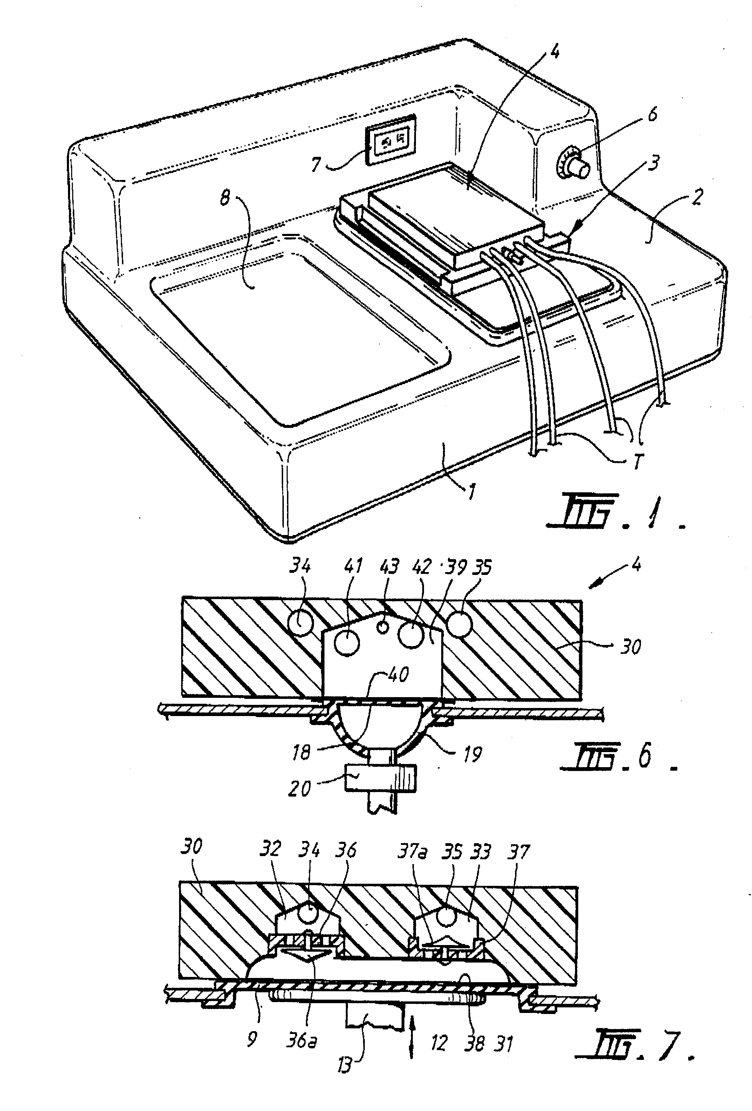

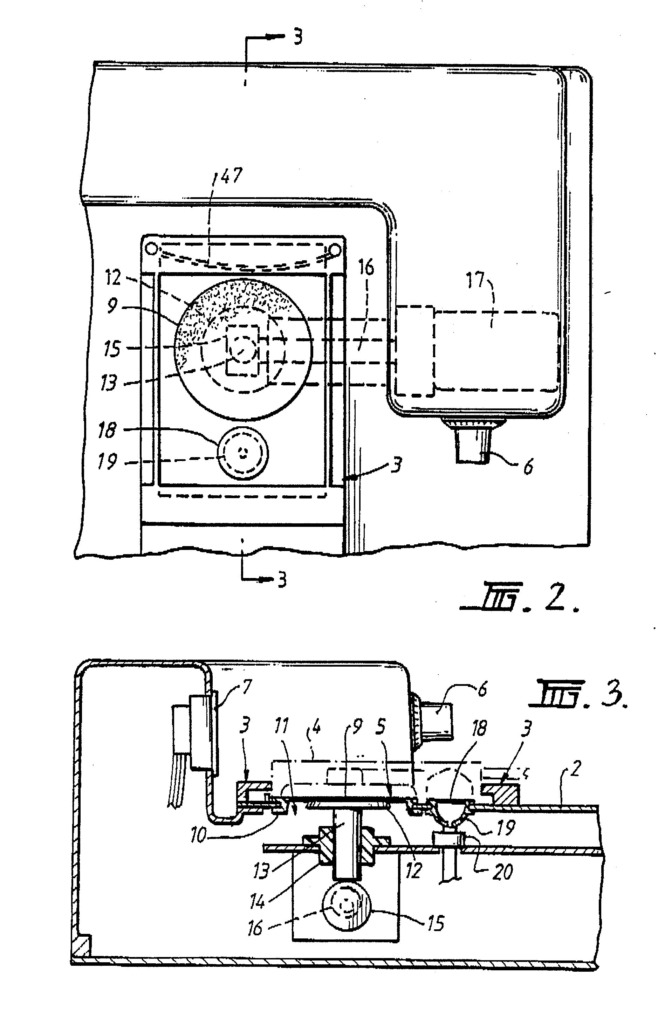

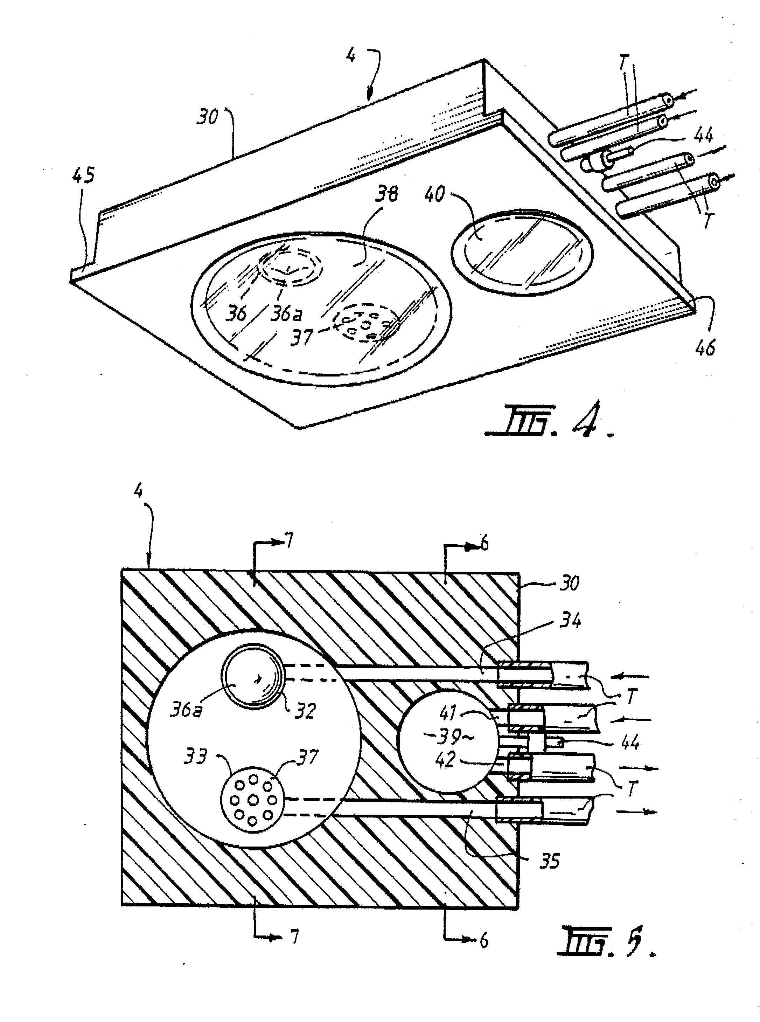

Referring firstly to figures 1 to 3 of the drawings, the pump embodying the invention will be seen to comprise a casing 1 having a generally flat top panel 2 having means 3 for securing a disposable pump element 4 embodying the invention in position over the pumping mechanism 5 (Fig. 3) of the pump, and having a pump speed varying knob 6, a pressure dial 7 and a recess 8 in the top panel 2 for receiving a tray for disposables such as syringes, plastic tubing and the like.

The pumping mechanism 5 comprises a flexible rubber diaphragm 9 formed with a peripheral slot 10 which engages with the edge of the top panel 2 surrounding a circular opening 11 which is formed in the top panel 2. A flat circular plate 12 carried by an upstanding stem 13 supported by a bearing 14 is disposed immediately below the diaphragm 9 and is reciprocated to distend the diaphragm 9 by means of an off-centre mounted roller 15 carried by a shaft 16 driven by a motor 17, the speed of which is controlled by the knob 6 connected to a rheostat (not shown).

Adjacent the diaphragm 9 is a further pressure sensing diaphragm 18 supported in an opening in the top panel 2 and closing a liquid filled chamber 19 connected to a pressure transducer 20 of any suitable type, such as a Motorola MPX 3100D, which is in turn connected to the pressure guage 7.

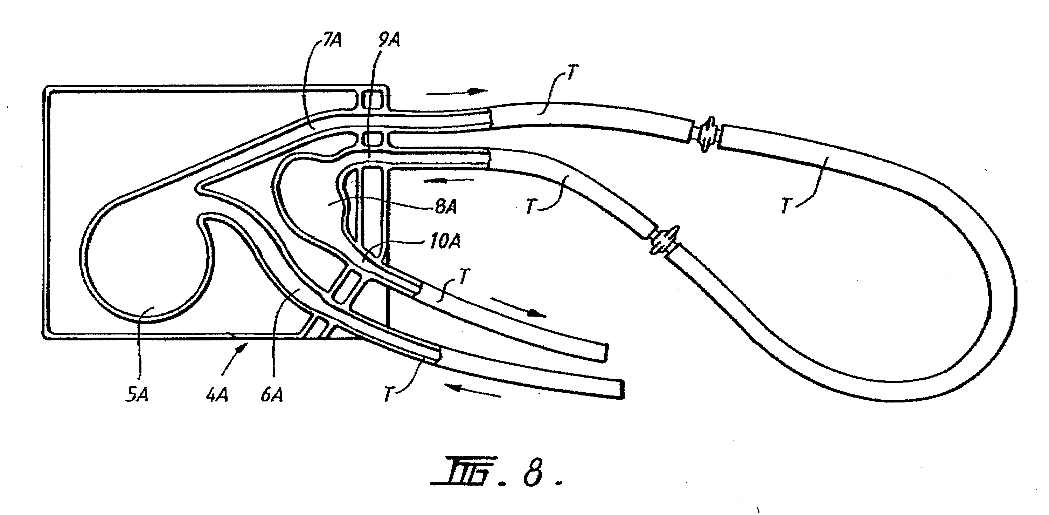

Referring now to figures 4 to 7 of the drawings, the disposable pump element embodying the invention will be seen to comprise an injection moulded plastics (such as transparent ABS resin) body 30 formed with a generally circular pumping chamber 31 having a pair of valve housings 32 and 33 into which inlet and outlet passages 34 and 35 open. The passages 34 and 35 extend through the body 30 to one end and the passages are enlarged adjacent the side of the body 30 to receive plastics tubing T of an internal diameter similar to the diameter of the passages 34 and 35. The tubing T is secured in the passages by solvent bonding, or by some other suitable means, so that the pump element 4 is supplied to the user with the tubing T attached.

One way valves 36 and 37 having centrally secured valve flaps 36a and 37a, moulded from a suitable polymer, such as C-flex, manufactured by Concept Polymers, covering an apertured valve body are received in the valve housings 32 and 33 to allow the passage of blood to and from the pumping chamber 31 in one direction only. It will be noted that the valve flaps 36a and 37a of the valves 36 and 37 are conically shaped at their outwardly directed surfaces, and this shaping serves to reduce the build up of air bubbles arounds the valves 36 and 37. The pumping chamber 31 is hermetically sealed by means of a flexible plastics wall 38 of transparent medical grade PVC, which is heat sealed to the face of the body 30 around the pumping chamber 31.

The body 30 is also formed with a pressure sensing chamber 39 closed by a wall 40 of transparent medical grade PVC, which is similarly heat sealed to the body 30 surrounding the chamber 39. Inlet and outlet passages 41 and 42 are formed in the body 30 and open into the chamber 39. Again plastics tubing T are fitted to the enlarged end portions of the passages 41 and 42, and secured as described above. The plastics tubing T in use connects inlet passage 34 to the patient, the outlet passage 35 to a dialysis machine, the outlet of which is connected to the inlet passage 41 to the pressure chamber 39, the outlet passage 42 of which is again connected to the patient to complete the circuit. To allow for more rapid initial priming of the pump, an air bleed passage 43 opens into the pressure chamber 39 and is closed by a suitable stop valve 44.

To enable one-handed insertion of the pump element 4 into engagement with the retaining means 3, the body 30 is formed with outwardly extending end flanges 45 and 46 which engage under the retaining means 3 against the action of a leaf spring 47 positioned within the front slot defined by the forward most retaining means 3. Thus, in use the operator simply inserts the front flange 45 under the front retaining means 3 and pushes forwardly against the leaf spring 47 until the rear flange 46 is able to engage the rear retaining means 3 to positively locate the pump element in position.

When the pump element 4 is located in position, it will be appreciated from figures 6 and 7 of the drawings that the diaphragm 9 is aligned with the flexible wall 38 of the pumping chamber 31 while the flexible element 18 of the pressure sensing member is located in alignment with the flexible wall 40 of the pressure sensing chamber 39. Thus, as the plate 12 is reciprocated by the roller 15 under the diaphragm 9, the flexible wall 38 is gently forced into the pump chamber 31 to displace approximately 90% of the blood which has filled the chamber, through the one way valve 37 into the outlet passage 35. Thus, the plate 12 distends the diaphragm 9 and gently forces the flexible wall 38 inwardly to enable the blood in the chamber 31 to be pumped in a gently pulsating manner somewhat similar to the action of a heart. Flow of blood within the chamber 31 and outlet passage 35 is generally laminar and accordingly the red cells in the blood are not adversely affected by the pumping operation. Since the pump element 4 is disposable, it is replaced on each occasion a pumping operation is required, which reduces the necessity for cleaning of the pump, thereby reducing the possibility of infection. The pump element 4 and the attached tubes are of course pre-sterilized in any suitable manner and are contained before use in a sterile package.

It will be appreciated that the diaphram 9 primarily acts as a cover for the plate 12 and is therefore not essential to the operation of the pump. However, for cleaning purposes, and to prevent damage to the flexible wall 38, it is desirable for a cover similar to the diaphragm 9 to be provided.

While the pump element 4 described above is preferred, similar results may be achieved by the use of a flexible pump element of the type shown in figure 8 of the drawings. In this embodiment, the flexible pump element 4A comprises a pair of medical quality PVC sheets heat welded together around the periphery to define a first flexible blood receiving chamber 5A, an inlet passage 6A adapted to receive a length of flexible tubing T and an outlet passage 7A similarly adapted to receive a length of plastics tubing T. A pressure monitoring chamber 8A is similarly defined by heat welding and is similarly provided with inlet and outlet passages 9A and 10A adapted to receive lengths of plastics tubing T. In each case, the tubing T is preferably heat sealed in position to ensure that it cannot be disconnected from its respective passage.

It will be appreciated that this embodiment of the pump element operates similarly to the previous embodiment since the PVC sheets defining the chambers 5A and 8A are able to flex in a manner similar to the flexible walls 38 and 40. However, principal difference is that this pumping element requires a reaction member to be secured in position over the pump element 4A so that the diaphragm 9 has a surface against which it may react. The reaction surface may for example be provided by a clear plastics plate adapted to engage the retaining means 3 in a manner similar to the pump element 4 and provided with depressions aligned with the pumping and pressure sensing chambers 5A and 8A to allow for the collection of a volume of blood in these chambers. To ensure positive location of the pump element 4A, location holes H may be provided to engage pins (not shown) secured to the top panel 2 of the pump casing 1.

The pressure monitoring sensing means 18, 19, 20 described above may be replaced by other commercially available sensors which do not require liquid to transfer the pressure from the pumped fluid to the transducer 20. Such an arrangement has the advantage that the transducer directly contacts the flexible wall 40.

A still further mechanism for achieving pumping in accordance with the invention is described in the provisional specification accompanying our Australian Patent Application PI 0939, the contents of which are incorporated into this specification by cross-reference.

The entire contents of the provisional specifications lodged with Australian Patent Applications of which this is the complete specification are hereby imported into this specification and form part of the disclosure of this specification. The claims form part of the disclosure of this specication.

**Claims**

1. A disposable pump element (4) for a pumping system comprising a pumping chamber (31) closed by a flexible or displaceable wall (38), inlet and outlet passages (34, 35) connected to said chamber, a pressure monitoring chamber (39) closed by a flexible or displaceable wall (40), and inlet and outlet passages (41, 42) opening into said monitoring chamber, said outlet passage (34) of said pumping chamber (31) being connectable to said inlet passage (41) of said monitoring chamber (39) so that the pressure of the fluid leaving said pumping chamber (31) is able to be monitored through said wall (40) closing said monitoring chamber (39) while maintaining the fluid being pumped within a closed system.

2. The pump element of claim 1, wherein said chambers (31, 39) and said passages (34, 35, 41, 42) are formed in a relatively rigid plastics moulding (30), said chambers (31, 39) being closed by one or more sheets (38, 40) of flexible plastics material secured to said moulding (30) to cover said chambers (31, 39).

3. The pump element of claim 1 or 2, wherein said moulding (30) supports one way valves (36, 37) in said inlet and outlet passages (34, 35) to said pumping chamber (31), said one way valves (36, 37) being positioned to prevent flow of fluid in the wrong direction.

4. The pump element of any preceding claim, wherein said moulding (30) is formed with means (45, 46) for enabling releasable attachment of said pump element to a pumping system.

5. The pump element of claim 1, comprising two flexible plastic films (Fig. 8) secured together to define said chambers (5A, 8A) and said passages (6A, 7A, 9A, 10A), said passages being adapted to receive plastics tubing (T) for the delivery of fluid to and from said pumping chamber (5A) and to and from said pressure monitoring chamber (8A).

6. A pumping system for blood and other fluids comprising a disposable pump element (4, 4A) according to any preceding claim together with pumping means (12) for causing generally pulsatile deflection of said flexible or displaceable wall (38) closing said pumping chamber (31) to cause displacement of fluid contained in said pumping chamber (31) through said outlet (35) passage and into said pressure monitoring chamber (39), and pressure monitoring means (18, 19, 20) positioned to be held in contact with said flexible or displaceable wall (40) of said pressure monitoring chamber (39) to sense the pressure of the fluid contained in said monitoring chamber (39), said pumping system including means (3) for detachably securing said disposable pump element (4) with its flexible or displaceable wall (38) in contact with said pumping and pressure monitoring means.

7. The pumping system of claim 6, wherein said pumping means comprises a plate (12) mounted for reciprocating movement into and out of said pumping chamber (31) in contact with said flexible or displaceable wall (38).

8. The pumping system of claim 7, wherein said plate (12) is covered by a flexible diaphragm (9) to reduce the risk of damage to said flexible or displaceable wall (38) and to facilitate cleaning of the pumping system.

9. The pumping system of any one of claims 6, 7 or 8, wherein said pressure monitoring means comprises a hollow body (19) containing incompressible liquid closed by a diaphragm (18) adapted to contact the flexible or displaceable wall (40) of said monitoring chamber (39), and a pressure transducer (20) connected to said hollow body (19) to sense changes in pressure in said liquid caused by changes in pressure of the fluid contained in said pressure monitoring chamber (39).

0 288 145

FIG. 1.

FIG. 6.

FIG. 7.

_Fig_. 2.

_Fig_. 3.

FIG. 4.

FIG. 5.

_Ⅱ**G**. 8._

0 288 145

## DOCUMENTS CONSIDERED TO BE RELEVANT

EP 88302322.8

| Category | Citation of document with indication, where appropriate, of relevant passages | Relevant to claim | CLASSIFICATION OF THE APPLICATION (Int. Cl 4) |
|---|---|---|---|
| Y | GB - A - 2 176 717 (COBE LAB.) | 1-4,6 | A 61 M 1/10 |
| A | * Fig. 1-5; page 1, lines 4-13; page 1, line 123 - page 2, line 17; page 2, lines 22-48; page 2, lines 87-103; page 3, lines 25-59 * | 9 | A 61 M 1/14 |
| | | | A 61 M 1/00 |
| | | | F 04 B 43/00 |
| | -- | | |
| Y | EP - A1 0 134 436 (GAMBRO LUNDIA) | 1-4,6 | |
| A | * Fig. 1,3,12,15; page 4, line 29 - page 5, line 8; page 5, line 29 - page 6, line 22; page 9, line 14 - page 11, line 18 * | 9 | |
| | -- | | |
| D,Y | US - A - 4 468 222 (I.H.LUNDQUIST) | 1-4,6 | |
| A | * Fig. 2,6,7,10,11; column 7, line 4 - column 8, line 20; column 8, line 61 - column 9, line 23; column 12, lines 39-64; claim 1 * | 7,8 | TECHNICAL FIELDS SEARCHED (Int. Cl 4) |
| | -- | | A 61 M 1/00 |
| D,A | US - A - 4 410 322 (G.K.ARCHIBALD) | 1-7,9 | A 61 M 5/00 |
| | * Totality * | | F 04 B 9/00 |
| | -- | | F 04 B 13/00 |
| A | US - A - 4 364 386 (J.A.JENKINS et al.) | 1,2,9 | F 04 B 43/00 |
| | | | F 04 B 39/00 |
| | * Fig. 1,2,6,7; column 3, line 26 - column 5, line 9; column 5, lines 27-62 * | | F 04 B 51/00 |
| | -- | | |
| A | US - A - 4 411 603 (M.J.KELL) | 1-4 | |
| | * Fig.; column 1, lines 6-11; column 4, lines 10-45; column 5, lines 20-23 * | | |
| | -- | | |

The present search report has been drawn up for all claims

| Place of search | Date of completion of the search | Examiner |
|---|---|---|
| VIENNA | 05-08-1988 | LUDWIG |

| | DOCUMENTS CONSIDERED TO BE RELEVANT | | EP 88302322.8 |
|---|---|---|---|

| Category | Citation of document with indication, where appropriate, of relevant passages | Relevant to claim | CLASSIFICATION OF THE APPLICATION (Int Cl 4) |
|---|---|---|---|
| A | <u>WO - A1 - 86/07 266</u> (WARNER LAMBERT) <br> * Fig. 1,2; page 4, line 18 - page 5, line 17 * <br> ---- | 1 | |
| | | | TECHNICAL FIELDS SEARCHED (Int. Cl 4) |

The present search report has been drawn up for all claims

| Place of search | Date of completion of the search | Examiner |
|---|---|---|
| VIENNA | 05-08-1988 | LUDWIG |